# EUROPEAN PATENT APPLICATION

(11) **EP 4 570 241 A1**
(43) Date of publication of application: **18.06.2025**
(21) Application number: 23386133.5
(22) Date of filing: 12.12.2023
(51) Int. Cl.: A61K 9/20, A61K 31/47

(54) **COMPOSITIONS OF IVACAFTOR**

(71) Applicant: Genepharm S.A., 15351 Pallini (GR)
(72) Inventor: Xenogiorgis, Vasileios, 17676 Kallithea, Athens (GR); Mpenekis, Vasilis, 11475 Athens (GR); Bekyrou, Chrysanthi, 15121 Pefki (GR)
(74) Representative: Haseltine Lake Kempner LLP

(57) **Abstract**

The present invention relates to a solid composition of amorphous ivacaftor wherein the composition is free from pH dependent polymers and a process for the preparation of such solid compositions.

## Description

### FIELD OF THE INVENTION

The present invention relates to a solid composition of amorphous ivacaftor wherein the composition is free from pH dependent polymers and a process for the preparation of such solid compositions.

### BACKGROUND OF THE INVENTION

ivacaftor, which is chemically known as N-(2,4-di-tert-butyl-5-hydroxyphenyl)-I, 4- dihydro-4-oxoquiniline-3-carboxamide is represented structurally by Formula (I) below:

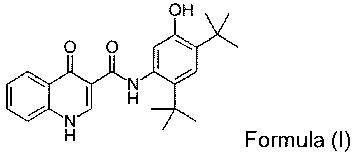

ivacaftor is a medication used to treat cystic fibrosis. Specifically, ivacaftor is used to treat people with certain mutations in the cystic fibrosis transmembrane conductance regulator (CFTR) gene, primarily the G551D mutation. These people account for about 4 to 5% of cystic fibrosis cases.

Cystic fibrosis is a genetic disorder that affects chloride transport throughout the epithelial cells of the body. Cystic fibrosis is caused by defects in the CFTR protein, which regulates fluid flow within cells and affects the components of sweat, digestive fluids, and mucus. Cystic fibrosis therefore can cause problems with the respiratory, endocrine, gastrointestinal, and reproductive systems.

Ivacaftor is a CFTR potentiator. Ivacaftor works by improving the function of the protein in the body to decrease the build-up of mucus in the lungs. Specifically, ivacaftor improves the transport of chloride in the abnormal CFTR protein by directly binding to ion channels to induce a non-conventional mode of gating, which in turn increases the probability that the ion channels are open.

Ivacaftor is commonly used in the crystalline form, as although amorphous ivacaftor is more soluble, amorphous ivacaftor is less thermodynamically stable and so converts to the crystalline form over time.

Often, to compensate for the lack of stability of amorphous ivacaftor, amorphous ivacaftor is formulated as a solid dispersion within a polymer matrix. There are different ways to produce solid dispersions, hot-melt extrusion and spray-drying are amongst the most commonly used techniques commercially. Both techniques have disadvantages such as ivacaftor degradation at high temperatures, limited polymer matrix choices, expensive and bulky technology and solid dispersions can result in compositions with low bulk density and very poor flow properties.

European Patent 2328618 discloses a pharmaceutical composition comprising a solid dispersion of amorphous ivacaftor with a polymer selected from hydroxypropyl methylcellulose (HPMC), Hypromellose cellulose Acetate Succinate (HPMCAS), vinylpyrrolidone/vinyl acetate copolymer (PVP/VA), polyvinylpyrrolidone (PVP), hydroxypropyl cellulose (HPC), methacrylic acid/methacrylate copolymers or any combination thereof.

PCT publication WO2022013360A1 discloses a pharmaceutical composition comprising amorphous ivacaftor without the use of a solid dispersion. However, the pharmaceutical composition requires the use of a pH dependent polymer as well as surfactant intragranularly and extragranularly to keep the amorphous form stable. The composition was also granulated using a slugging process to retain the amorphous form of ivacaftor without the use of solid dispersion technique. The slugging process is very time and energy consuming, due to the additional process steps and excipients required.

It is therefore desirable to provide alternative compositions of amorphous ivacaftor. In particular, it is desirable to provide amorphous ivacaftor compositions that are stable and/or cost effective.

### SUMMARY OF THE INVENTION

The present invention is defined in the appended claims.

In accordance with a first aspect, there is provided a solid composition of amorphous ivacaftor wherein the composition is free from pH dependent polymers.

The term "free from pH dependent polymers" as used herein refers to compositions where pH dependent polymers have not been added to a composition. For example, "free from pH dependent polymers" means that a pH dependent polymer is present in the composition in an amount of less than about 1.0 wt.%, or less than about 0.5 wt.%, or less than about 0.3 wt.%, or less than about 0.2 wt.%, or less than about 0.1 wt.%, or less than about 0.05 wt.%, or less than about 0.01 wt.%, or less than about 0.005 wt.% based on the total weight of the solid composition.

The inventors surprisingly found that a solid composition according to the present invention may exhibit favourable physical attributes sufficient for manufacturing, alongside sufficient stability and/or dissolution when compared to a current commercial product. Examples of the present invention were shown to display comparable dissolution and/or assay performance and/or stability when compared to solid compositions with pH dependent polymers.

In accordance with a second aspect, there is provided a process for the preparation of a solid composition according to the first aspect.

In embodiments, the solid composition is prepared using roller compaction.

Roller compaction may be beneficial as it does not require additional lubricant, unlike the dry granulation process of slugging, nor is it as energy intensive.

Certain embodiments of the present invention may provide one or more of the following advantages:
- desired stability of amorphous ivacaftor compositions;
- desired cost of amorphous ivacaftor compositions;
- desired ease of handling of amorphous ivacaftor compositions;
- desired ease of preparation of amorphous ivacaftor compositions.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE DRAWINGS

The invention will further be illustrated by reference to the following figures:

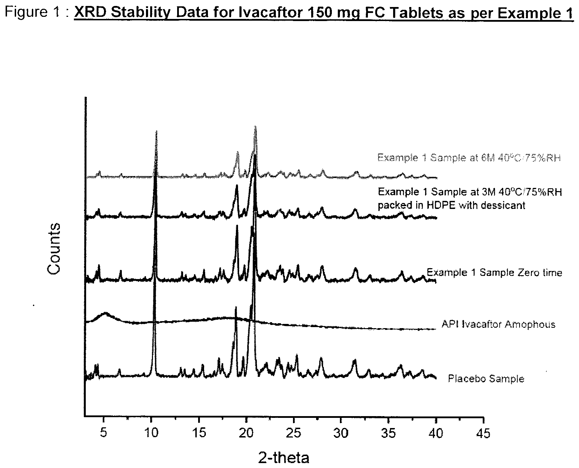

Figure 1: shows an XRD spectrum illustrating the stability of ivacaftor 150 mg Tablets formulated according to the present invention.

It is understood that the following description and references to the figures concern exemplary embodiments of the present invention and shall not be limiting the scope of the claims.

### DETAILED DESCRIPTION

The present invention is based on the surprising finding that amorphous ivacaftor compositions with good stability can be obtained without the presence of a pH dependent polymer.

A pH dependent polymer is a polymer which responds to changes in the pH of a surrounding medium by varying its solubility.

As used herein, the term "amorphous" refers to a solid material having no long range order in the position of its molecules. Amorphous solids cannot be defined by a finite unit cell. For example, unlike crystalline materials which exhibit strong Bragg diffraction, the X-Ray Diffraction patterns of amorphous materials are characterized by broad and diffuse peaks.

When ranges are used herein, all combinations and sub-combinations of ranges and specific embodiments therein are intended to be included.

As used herein, the term "about" when referring to a number or a numerical range means that the number or numerical range referred to is an approximation within experimental variability (or within statistical experimental error), and thus the number or numerical range may vary. Typical experimental variabilities may stem from, for example, changes and adjustments necessary during scale-up from laboratory experimental and manufacturing settings to large scale, GMP manufacturing conditions as is known to those familiar with the art of pharmaceutical development and manufacturing. Such changes can vary between 1% and 10% of the stated number or numerical range.

As used herein, the term "comprising" (and related terms such as "comprise" or "comprises" or "having" or "including") has an open meaning and therefore a pharmaceutical composition comprising described features may comprise additional components in addition to the described features.

Abbreviations used herein have their conventional meaning within the chemical and biological arts, unless otherwise indicated.

As used herein, the term "dispersion" generally refers to a system where a substance is dispersed within another substance.

As used herein, the term "solid dispersion" generally refers to the solid-state dispersion of one or more active substances in an inert carrier excipient, for example, one or more poorly soluble drugs within a water-soluble polymer.

As used herein, the term "excipient" refers to an inactive ingredient in a composition.

As used herein, the term "diluent" or "filler" refers to an excipient that adds bulk to a composition.

As used herein, the term "disintegrant" refers to an excipient that promotes disintegration of a tablet/composition into smaller fragments in an aqueous environment.

As used herein, the term "surfactant" refers to an excipient that decreases surface or interfacial tension to impart compositions with enhanced solubility and/or wettability.

As used herein, the term "binder" refers to an excipient that improves cohesion, and plasticity of a composition.

As used herein, the term "treatment" refers to an approach for obtaining beneficial or desired results including, but not limited to, therapeutic benefit and/or a prophylactic benefit. The term "patient" refers to an animal, such as a mammal, for example a human.

As used herein, the term "intragranular" refers to a component added before a granulation process.

As used herein, the term "extragranular" refers to a component added after granulation and before a compression process.

In certain embodiments the solid composition according to the present invention comprises amorphous ivacaftor in an amount of from about 15 wt.% to about 40 wt.% based on the total weight of the solid composition. In some embodiments, for example, the solid composition according to the present invention comprises amorphous ivacaftor in an amount of from about 15 wt.% to about 35 wt. %, from about 20 wt.% to about 40 wt. %, from about 25 wt.% to about 35 wt.%, or from about 27 wt.% to about 30 wt.%. In some embodiments, for example, the solid composition according to the present invention comprises amorphous ivacaftor in an amount of about 27 wt.%

In certain embodiments, the solid composition according to the present invention comprises amorphous ivacaftor wherein the amorphous ivacaftor has a d₉₀ particle size, as determined by laser light diffraction, of about 110 µm or less, for example, of about 100 µm or less, of about 95 µm or less, of about 85 µm or less, of about 75 µm or less, of about 65 µm or less of about 55 µm or less, of about 45 µm or less, of about 35 µm or less, of about 25 µm or less, of about 20 µm or less, of about 15 µm or less, of about 10 µm or less, or of about 7.5 µm or less.

The d₉₀ particle size of the ivacaftor is measured in a well-known manner by a laser light diffraction according to the method of Chapter 2.9.31 of the European Pharmacopoeia, 11th Edition (2023). A Malvern Mastersizer (3000), Hydro-3000MV (accessory) and Analytical balance were used for d₉₀ particle size determination. Particle size is determined by passing a laser beam through a dispersed sample and measuring the variation in angular scattered light intensity. The median particle size d₉₀ is the value at which there are 90% by number of particles which have an equivalent spherical diameter less than that d₉₀ value.

In certain embodiments, the pH dependent polymer as disclosed herein is selected from hypromellose acetate succinate (HPMCAS), hydroxypropyl methylcellulose (HPMC), cellulose acetate phthalate (CAP), polymethacrylates, hydroxypropyl methyl cellulose phthalates (HPMCP), carboxymethylcellulose (CMC), salts of carboxymethylcellulose, cellulose acetate trimellitate (CAT), hydroxypropylcellulose acetate phthalate (HPCAP), hydroxypropylmethyl-cellulose acetate phthalate (HPMCAP), and methylcellulose acetate phthalate (MCAP), and combinations thereof. In some embodiments, the pH dependent polymer is HPMCAS.

In certain embodiments, a solid composition of amorphous ivacaftor further comprises a surfactant, a filler/diluent, a disintegrant, a glidant and/or a lubricant.

In certain embodiments, the solid composition of amorphous ivacaftor comprises a surfactant selected from sodium lauryl sulfate, polyethylene oxide)-poly(propylene oxide)-poly(ethylene oxide), poloxamer, polysorbate 20 and polysorbate 80, or a combination thereof. In some embodiments, the solid composition comprises surfactant in an amount of about 0.1 wt.% to about 5 wt.%, for example, of from about 0.1 wt.% to about 4 wt.%, of from about 0.5 wt.% to about 3 wt.%, or of from about 1 wt.% to about 2 wt.%. In some embodiments, for example, the solid composition according to the present invention comprises a surfactant in an amount of about 2 wt.%.

In certain embodiments, the solid composition is free from intragranular lubricant. The term "free from intragranular lubricant" as used herein refers to compositions where lubricant has not been added to a composition to achieve a particular effect prior to granulation. For example, "free from intragranular lubricant" means that an intragranular lubricant is present in the composition in an amount of less than about 0.5 wt.%, or less than about 0.3 wt.%, or less than about 0.2 wt.%, or less than about 0.1 wt.%, or less than about 0.05 wt.%, or less than about 0.01 wt.%, or less than about 0.005 wt.% based on the total weight of the solid composition.

In certain embodiments, the solid composition of amorphous ivacaftor comprises a filler or diluent selected from silicified microcrystalline cellulose, microcrystalline cellulose, mannitol, starch, pregelatinized starch and lactose, or a combination thereof. In some embodiments, the lactose may be lactose anhydrous. In alternative embodiments, the lactose may be lactose monohydrate. In some embodiments, the solid composition comprises filler or diluent in an amount of about 30 wt.% to about 70 wt.%, for example, from about 30 wt.% to about 65 wt.%, from about 35 wt.% to about 60 wt.%, from about 40 wt.% to about 55 wt.%, or from about 45 wt.% to about 50 wt.%. In certain embodiments, the solid composition comprises filler or diluent in an amount of about 55 wt.% to about 65 wt.%.

In certain embodiments, the solid composition of amorphous ivacaftor comprises a disintegrant selected from pregelatinized starch, croscarmellose sodium and crospovidone, or a combination thereof. In some embodiments, the solid composition comprises disintegrant in an amount of about 1 wt.% to about 10 wt.%, for example, from about 1 wt.% to about 9 wt.%, from about 2 wt.% to about 8 wt.%, from about 3 wt.% to about 7 wt.%, or from about 4 wt.% to about 6 wt.%. In certain embodiments, the solid composition comprises disintegrant in an amount from about 5 wt.% to about 8 wt.%.

In certain embodiments, the solid composition of amorphous ivacaftor comprises a glidant selected from colloidal silica, fumed silica, talc and magnesium carbonate, or a combination thereof. In some embodiments, the solid composition comprises glidant in an amount of about 0.1 wt.% to about 5 wt.%, for example, from about 0.5 wt.% to about 4.5 wt.%, from about 1 wt.% to about 4 wt.%, or from about 1 wt.% to about 3 wt.%. In some embodiments, for example, the solid composition according to the present invention comprises a glidant in an amount of about 2 wt.%.

In certain embodiments, the solid composition of amorphous ivacaftor comprises a lubricant selected from magnesium stearate and sodium stearyl fumarate, or a combination thereof. In some embodiments, the solid composition comprises lubricant in an amount of from about 0.1 wt.% to about 5 wt.%, for example, from about 0.5 wt.% to about 4.5 wt.%, from about 1 wt.% to about 4 wt.%, or from about 2 wt.% to about 4 wt.%. In some embodiments, for example, the solid composition according to the present invention comprises a lubricant in an amount of about 3 wt.%.

In certain embodiments, the solid composition of amorphous ivacaftor is free from solid dispersions. Solid dispersions may be prepared by mixing ivacaftor with a polymer and subjecting the mixture to spray drying or hot melt extrusion process. Hot-melt extrusion is a manufacturing process where polymeric materials are heated above their glass-transition temperature and mechanically mixed with active compounds in order to disperse the drug substance molecularly into the polymeric net. Spray -drying consists in two main steps: i) complete dissolution of the drug substance and a dilution material (e.g., a polymer) in a solvent or mixture of solvents and ii) fast spray -drying of the previous solution in order to collect the solid material. The polymer may be selected from HPMC, HPMCAS, PVP, HPC, methacrylic acid/methacrylate copolymers or any combinations thereof.

Processes such as spray drying, or hot melt extrusion are time intensive and require expensive equipment. Furthermore, spray drying commonly involves dissolving the drug substance and the excipients in a solvent, which may lead to unwanted chemical reactions, and hot melt extrusion commonly requires high temperatures so active compounds may degrade. As such, a composition free from solid dispersions may be advantageous.

In certain embodiments, the solid composition of amorphous ivacaftor is a free-flowing powder, granule or a tablet.

In certain embodiments, the solid composition of amorphous ivacaftor comprises:
(a) amorphous ivacaftor in an amount of about 15 wt.% to about 40 wt.%;
(b) a surfactant in an amount of about 0.1 wt.% to about 5 wt.%;
(c) a filler or a diluent in an amount of about 30 wt.% to about 70 wt.%;
(d) a disintegrant in an amount of about 1 wt.% to about 10 by wt.%;
(e) a glidant in an amount of about 0.1 wt.% to about 5 wt.%;
(f) a lubricant in an amount of about 0.1 wt.% to about 5 wt.%;
wherein each component is based on the total weight of the solid composition.

In certain embodiments, the solid composition of amorphous ivacaftor comprises:
(a) amorphous ivacaftor in an amount of about 20 wt.% to about 30 wt.%;
(b) a surfactant in an amount of about 1 wt.% to about 2 wt.%;
(c) a filler or a diluent in an amount of about 50 wt.% to about 65 wt.%;
(d) a disintegrant in an amount of about 4 wt.% to about 8 wt.%;
(e) a glidant in an amount of about 1 wt.% to about 4 wt.%;
(f) a lubricant in an amount of about 1 wt.% to about 4 wt.%;
wherein each component is based on the total weight of the solid composition.

In certain embodiments, the solid composition of amorphous ivacaftor comprises:
(a) amorphous ivacaftor in an amount of about 25 wt.% to about 30 wt.%;
(b) a surfactant in an amount of about 1 wt.% to about 2 wt.%;
(c) a filler or a diluent in an amount of about 50 wt.% to about 65 wt.%;
(d) a disintegrant in an amount of about 4 wt.% to about 8 wt.%;
(e) a glidant in an amount of about 1 wt.% to about 4 wt.%;
(f) a lubricant in an amount of about 1 wt.% to about 4 wt.%;
wherein each component is based on the total weight of the solid composition.

In certain embodiments, the solid composition of amorphous ivacaftor comprises:
(a) amorphous ivacaftor in an amount of about 25 wt.% to about 30 wt.%;
(b) a surfactant in an amount of about 1.5 wt.% to about 2.5 wt.%;
(c) a filler or a diluent in an amount of about 55 wt.% to about 65 wt.%;
(d) a disintegrant in an amount of about 5 wt.% to about 8 wt.%;
(e) a glidant in an amount of about 1 wt.% to about 3 wt.%;
(f) a lubricant in an amount of about 2 wt.% to about 4 wt.%;
wherein each component is based on the total weight of the solid composition.

In certain embodiments, the solid composition of amorphous ivacaftor comprises:
(a) amorphous ivacaftor in an amount of about 27 wt.%;
(b) a surfactant in an amount of about 2 wt.%;
(c) a filler or a diluent in an amount of about 61 wt.%;
(d) a disintegrant in an amount of about 5 wt.%;
(e) a glidant in an amount of about 2 wt.%;
(f) a lubricant in an amount of about 3 wt.%;
wherein each component is based on the total weight of the solid composition.

In certain embodiments, the solid composition of amorphous ivacaftor comprises:
(a) amorphous ivacaftor in an amount of about 27 wt.%;
(b) a surfactant in an amount of about 2 wt.%;
(c) a filler or a diluent in an amount of about 57.5 wt.%;
(d) a disintegrant in an amount of about 8 wt.%;
(e) a glidant in an amount of about 2 wt.%;
(f) a lubricant in an amount of about 3 wt.%;
wherein each component is based on the total weight of the solid composition.

In certain embodiments, the solid composition of amorphous ivacaftor is coated.

In certain embodiments, the solid composition of amorphous ivacaftor is coated with a polymeric film coating material. Numerous suitable polymeric film coating materials are known in the art. In certain embodiments, the polymeric film coating material is selected from a film-coating polymer such as polyvinyl alcohol and/or polyethylene glycol. In some embodiments, the polymeric film-coating polymer is selected from hydroxypropylmethylcellulose or hydroxypropylcellulosef.

In certain embodiments, in addition to the polymeric film-coating polymer, the polymeric film coating material further comprises a plasticizer. In some embodiments, the plasticizer is selected from propylene glycol, and optionally a pigment such as titanium dioxide.

In some embodiments, the coating may be selected from Opadry^{®} II Blue or Kollicoat^{®}.

In certain embodiments, the polymeric film coating material may also include an anti-adhesive. In some embodiments, the anti-adhesive is talc.

In certain embodiments, the solid composition of amorphous ivacaftor is an immediate release composition.

As used herein, the term "immediate release" generally refers to a composition that is developed to dissolve without delaying or prolonging dissolution or absorption of the drug.

In certain embodiments, the immediate release composition according to the present invention has less than about 5 wt.% coating based on the total weight of the solid composition, for example, less than about 4 wt.% coating, less than about 3 wt.% coating, less than about 2 wt.% coating, less than about 1 wt.%, or less than about 0.5 wt.% coating based on the total weight of the solid composition.

In certain embodiments, the dissolution of ivacaftor from a tablet formed from the solid composition of the present invention is at least about 75 % when the tablet is subjected to dissolution tests at pH range from 1.2 to 6.8 at 37° C ± 0.5°C using a USPII paddle apparatus with stirring speed of 50 rpm for 30 minutes. For example, the dissolution of ivacaftor from a tablet formed from the solid composition of the present invention is at least about 80%, at least about 85%, or at least about 90% when the tablet is subjected to dissolution tests at a pH range from 1.2 to 6.8 at 37° C ± 0.5°C using a USPII paddle apparatus with stirring speed of 50 rpm for 30 minutes. Such dissolution is beneficial, as it shows the tablet has good bioavailability. A tablet formed from the solid composition of the present invention having a high percentage of dissolution may illustrate the rate at which the active ingredient would be released during treatment of a patient.

In certain embodiments, the solid composition of amorphous ivacaftor is stable for at least about 1 month, at least about 2 months, at least about 3 months, at least about 4 months, at least about 5 months, or at least about 6 months at about 40 °C and about 75% relative humidity. The term stable is defined herein with regards to polymorphic stability and/or chemical stability. The chemical stability may be measured by suitable methods such as X-ray diffraction (XRD), dissolution and High-Performance Liquid Chromatography (HPLC). For example, the amorphous nature and active purity of the solid composition of amorphous ivacaftor is retained at about 40 °C and about 75% relative humidity for at least about 3 months. For example, the amorphous nature and active purity of the solid composition of amorphous ivacaftor is retained at about 40 °C and about 75% relative humidity for at least about 6 months.

In certain embodiments, the tablets formed from the solid composition of the present invention comprise up to about 150 mg of ivacaftor in a single dose. The tablet compositions of ivacaftor are suitable for oral administration of ivacaftor as a treatment for a patient.

In certain embodiments, the solid composition is free from extragranular excipients.

In certain embodiments, the solid composition is prepared using roller compaction.

Roller compaction is beneficially a continuous process and so can lead to a production time reduction, and the scale-up may be considered easier than other dry granulation methods, therefore, production costs can be reduced, whilst overall production efficiency is improved.

In certain embodiments, the solid composition may have one or more of the following effects:
- increased simplicity of the composition;
- increased stability of the composition;
- maintenance of the stability of the composition;
- reduced cost of the composition;
- increased ease of preparing the composition.

### EXAMPLES

The following illustrates examples of the solid composition of amorphous ivacaftor, and related aspects described herein. Thus, these examples should not be considered to restrict the present disclosure, but are merely in place to teach how to carry out the present disclosure.

### Section A: Compositions and their methods of production

To test manufacturing suitability and feasibility, four examples were screened. Two examples were prepared according to the present invention. Two Comparative Examples were used. Comparative Example 1 comprises the pH dependent polymer, Hypromellose Acetate Succinate (HPMCAS). and Comparative Example 2 is the commercially available Kalydeco^{®} tablets, which also comprised HPMCAS and contained 150 mg ivacaftor.

Kalydeco^{®} is marketed as an amorphous solid dispersion of ivacaftor. Kalydeco^{®} is practically insoluble in water and in buffers of pH 1.0 to 7.0. Kalydeco^{®} avoids the stability problems of amorphous ivacaftor by including the pH dependent polymer HPMCAS, and formulating ivacaftor as a solid dispersion, wherein the amorphous form is stable, spray drying is also used to produce Kalydeco^{®}. The Kalydeco^{®} used was supplied, for example by Vertex Pharmaceuticals Inc., according to the data sheet available from the European Medicines Agency at the date of filing.

Each example was formed as a tablet containing 150 mg ivacaftor. The final core weight (before coating) of each example tablet was set to 550 mg.

Each example was prepared using dry granulation. Examples 1 and 2 were dry granulated using a roller compaction process, whilst Comparative Example 1 was dry granulated using a slugging process.

The compositions of the Examples 1, 2 and Comparative Example 1 are shown below in Table 1. The composition of the commercially available Kalydeco^{®} tablet, is shown in Table 2.

**Table 1 - Composition of 150 mg ivacaftor tablets as prepared**

| | Example 1 | Example 2 | Comparative Example 1 |
|---|---|---|---|
| **Components** | % **by weight** | % **by weight** | % **by weight** |
| **Intragranular** | **Intragranular** | **Intragranular** | **Intragranular** |
| Ivacaftor (Amorphous) | 27.27 | 27.27 | 27.27 |
| Sodium Laurilsulfate (SLS) | 2.00 | 2.00 | 1.00 |
| Mannitol 200SD | 8.00 | - | - |
| Lactose Anhydrous | 33.73 | 40.09 | 12.29 |
| Cellulose, Microcrystalline 102 (MCC 102) | 14.00 | - | 45.45 |
| Starch 1500 | 5.00 | - | - |
| Croscarmellose Sodium | 5.00 | 8.00 | 6.00 |
| Silica, Colloidal Anhydrous (Aerosil 200) | 2.00 | 2.00 | 0.50 |
| Silicified Microcrystalline Cellulose (Prosolv^{®} HD90) | - | 17.64 | - |
| Magnesium Stearate | - | - | 0.50 |
| Hypromellose Acetate Succinate (HPMCAS) | - | - | 6.48 |
| Lactose Monohydrate | - | - | 12.29 |

| **Lubrication** | **Lubrication** | **Lubrication** | **Lubrication** |
|---|---|---|---|
| Sodium Stearyl Fumarate (SSF) | 3.00 | 3.00 | - |
| Magnesium Stearate | - | - | 0.50* |

| **Coating** | **Coating** | **Coating** | **Coating** |
|---|---|---|---|
| Opadry^{®} II Blue | 2.00 | 2.00 | 2.00* |
| **Coated Tablet Weight** | 102.00 | 102.00 | 102.00* |

| | | | |
|---|---|---|---|
| ***Intended Composition Amount** | | | |

**Table 2 - Composition of Comparative Example 2 - Kalydeco^{®} 150 mg ivacaftor tablets**

| **Components** | **mg/ per tablet** |
|---|---|
| Ivacaftor | 150 |
| Lactose Monohydrate | 167.2 |
| Cellulose, Microcrystalline | N/A |
| Hypromellose Acetate Succinate (HPMCAS) | N/A |
| Croscarmellose Sodium | N/A |
| Sodium Laurilsulfate (E487) | N/A |
| Silica, Colloidal Anhydrous | N/A |
| Magnesium Stearate | N/A |
| Opadry^{®} II Blue | N/A |
| Opacode^{®} Black | N/A |

| | |
|---|---|
| N/A - denotes the quantity of the component is not unavailable, but the component is present. | |

### Method of Preparation for Example 1 and Example 2

Examples 1 and 2 according to the present invention were prepared according to the following steps:

### Step 1: Formation of Blend A

Each intragranular component was sieved through a 30-mesh sieve and mixed together in a bin mixer at 20 rpm to form Blend A.

### Step 2: Dry Granulation

Blend A from Step 1 was then dry granulated through a roller compactor according to specific parameters, detailed in Table 3 below, to form ribbons.

### Step 3: Sizing to form Blend B

The ribbons resulting from Step 2 were then crushed and passed through a 30-mesh sieve, and mixed to form Blend B.

### Step 4: Lubrication

The SSF lubricant was sieved through a 60-mesh sieve, and mixed with Blend B to form lubricated Blend C.

### Step 5: Compression

Blend C from Step 4 was compressed to form core tablets.

### Step 6: Coating

The compressed core tablets were coated with Opadry^{®} II Blue.

**Table 3 - Roller Compaction Parameters**

| **Parameter** | **Range** |
|---|---|
| Main Roll Speed (rpm) | 3 - 5 |
| Feed Screw Speed (rpm) | 10 - 150 |
| Roller Distance (mm) | 0.5 - 1.0 |
| Load on Main Roller (kg) | 80 - 250 |
| Number of Rollers | 2 |

### Method of Preparation of Comparative Example 1

Comparative Example 1 was intended to be prepared according to the following steps:

### Step 1: Formation of Blend A

The intragranular components (except for Magnesium Stearate) were sieved through a 30-mesh sieve and mixed to form Blend A

### Step 2: Formation of Blend B

Magnesium Stearate was sieved through a 40-mesh sieve, and mixed with Blend A to form Blend B.

### Step 3: Slugging

Blend B from Step 2 was then compressed using a Karnavati minipress-II DL 10MT.

### Step 4: Sizing

The resultant slugs from Step 3 were then crushed and sized through a 30-mesh sieve and mixed together.

### Step 5: Lubrication

The Magnesium Stearate lubricant was sieved through a 40-mesh sieve and mixed with the sized slugs from Step 4 to form a lubricated blend, Blend C.

### Step 6: Compression

Blend C from Step 5 was compressed to form core tablets.

### Step 7: Coating

The compressed core tablets from Step 6 were coated with Opadry^{®} II Blue.

Comparative Example 1 was prepared according to Steps 1 to 3 above. Steps 4 to 7 were unable to be completed due to manufacturing problems.

### Section B: Testing of Examples 1 and 2, and Comparative Examples 1 and 2

The physical attributes, the dissolution and assays of the Examples were tested. The powder flowability properties of Examples 1 and 2, and Comparative Example 1 were conducted. As Comparative Example 2 was a commercial comparative example, the test could not be conducted.

The powder flowability was measured using the method according to Chapter 2.9.36 of the European Pharmacopoeia, 11th Edition (2023). The flowability properties were assessed using Carr index (indicator of compressibility of powder) and Hausner ratio (flowability of a powder). The Carr index and/or Hausner ratio were tested by measuring bulk and tapped density of the power according to Chapter 2.9.34 of the European Pharmacopoeia, 11th Edition (2023). Bulk density was tested using a 250 ml volumetric cylinder with a test sample mass of 100 g without compacting. Tapped density was measured using a Copley Scientific Powder Density Tester, with a 250 ml volumetric cylinder with a test sample mass of 100 g without compacting.

As can be seen in Table 4 below, Examples 1 and 2 displayed good powder flowability properties (Carr index ratio lower than about 15, and a Hausner ratio lower than about 1.18). Comparative Example 1 displayed insufficient, very very poor flow properties (Carr index ratio greater than about 38, and a Hausner ratio greater than about 1.60).

The compression performance of Examples 1 and 2, and Comparative Example 2 was investigated. As above, as Comparative Example 2 was a commercial comparative example, the test could not be conducted. The compression performance was tested by compressing the lubricated blend to form tablets as detailed in Method Step 5 for Examples 1 and 2. As can be seen in Table 3 below, Examples 1 and 2 displayed good compressibility properties to form tablets.

The dry granulation of Comparative Example 1 during the slugging process was problematic. The slugging process resulted in slugs with increased and out-of-specification weight variation, alongside severe capping, and sticking problems. The rest of the manufacturing process (sizing and lubrication) could not be carried out and therefore, no further analytical results could be obtained. The compression performance of Comparative Example 1 was therefore unable to be tested, as core tablets were unable to be formed from a lubricated blend.

The average weight of Examples 1 and 2, and Comparative Example 2 was determined. The analysis was performed using a standard technique, weighing 20 tablets on an Analytical Balance supplied by Kern. Weight variation may be indicative of uniformity of the dosage unit, and may therefore be important for product safety, identity, and quality. It is beneficial to be able to tablet a composition quickly and consistently, such that the level of active ingredient in each tablet is within the specifications and does not vary significantly from tablet to tablet.

As can be seen in Table 4 below, the average weight showed little variation for Examples 1 and 2 and was comparable to the commercial Comparative Example 2.

The hardness of Examples 1 and 2, and Comparative Example 2 was analysed. The hardness of the tablets was analysed by testing the resistance of the tablets to crushing. A Varian Benchsaver Series VK 200 Tablet Hardness Tester 40-2012 was used. The apparatus consisted of two jaws facing each other, one of which moves towards the other. The flat surfaces of the jaws are perpendicular to the direction of movement. The crushing surfaces of the jaws are flat and larger than the zone of contact with the tablet. The apparatus was calibrated using a system with a precision of 1 newton according to Chapter 2.9.8 of the European Pharmacopoeia, 11th Edition (2023).

As can be seen in Table 4, the hardness for Examples 1 and 2 was comparable to Comparative Example 2. Hardness is often related to the quality of the tablet, with a higher value being potentially beneficial for manufacturing purposes.

The disintegration of Examples 1 and 2, and Comparative Example 2 was analysed. The disintegration was tested according to the methodology of Test A of Chapter 2.9.1 of the European Pharmacopoeia, 11th Edition (2023). Disintegration may be an indicator of the ability of a tablet to break down into smaller particles or granules to allow the active drug to be absorbed into the body. Examples 1 and 2 displayed similar disintegration to one another. The disintegration time displayed for Examples 1 and 2 was beneficially within specification limits.

**Table 4 - Physical Attributes**

| **Test** | **Example 1** | **Example 2** | **Comparative Example 1** | **Comparative Example 2** |
|---|---|---|---|---|
| Powder Flowability Properties | Good | Good | Very Very Poor | - |
| Compression Performance of Lubricated Blend | Good | Good | Not Possible to Form Tablets | - |
| Average Weight (mg) | 564.0 | 564.3 | - | 564.8 |
| Hardness (N) | 126.7 | 127.3 | - | 1489 |
| Disintegration time (min:sec) | 05:28 | 05:42 | - | 01:05 |

The dissolution of Examples 1 and 2, and Comparative Example 2 was analysed. The dissolution was tested according to the methodology of Chapter 2.9.3 of the European Pharmacopoeia, 11th Edition (2023). A paddled dissolution apparatus supplied by Distek was used, 900 mL of 50 mM Sodium Phosphate Buffer and 0.7% SLS was used, and the paddle speed was set to 50 rpm.

As can be seen in Table 5, Examples 1 and 2 displayed dissolution results similar to Comparative Example 2, the commercial comparison. A high dissolution rate is beneficial as may indicate good bioavailability and may correlate to the rate at which the active ingredient is released into the body. The dissolution times for both Examples 1 and 2 beneficially meet the acceptance criteria for immediate-release dosage forms.

**Table 5 - Dissolution**

| **Time (min)** | **Example 1** | **Example 2** | **Comparative Example 1** | **Comparative Example 2** |
|---|---|---|---|---|
| 10 | 76.0 | 80.0 | - | 78.6 |
| 15 | 84.1 | 86.8 | - | 88.6 |
| 30 | 90.6 | 90.5 | - | 94.2 |
| 45 | 91.5 | 91.0 | - | 95.2 |

Assay by HPLC of Examples 1 and 2, and Comparative Example 2 was conducted. HPLC is a commonly used method for assay of pharmaceutical substances, and was performed according to the methodology of Chapter 2.2.29 of the European Pharmacopoeia, 11th Edition (2023).

Assay by HPLC may be used as a quality control parameter, as it can be used to confirm the identity of a pharmaceutical substance. As shown in Table 6, examples 1 and 2 displayed good assay results, similar to Comparative Example 2, the commercial comparison.

**Table 6 - Assay**

| | **Example 1** | **Example 2** | **Comparative Example 1** | **Comparative Example 2** |
|---|---|---|---|---|
| **Assay** (%) | 99.2 | 98.2 | - | 102.3 |

### SECTION C: Stability Data for Ivacaftor 150mg FC Tablets of Example 1 and Comparative Example 2

Further stability testing was carried out based on the tablets of Example 1. In particular, the stability of the tablets was tested by measuring dissolution, assay HPLC, related substances and crystallinity. Unless mentioned otherwise, the techniques used were as noted above.

Example 1 was placed in high-density polyethylene, with desiccant, and stored at 40 °C and 75% relative humidity for 3 months. After the 3 months, the dissolution, assay and amount of related substances of Example 1 were analysed. The results of which are shown below in Tables 7 to 9.

**Table 7 - Dissolution Comparison**

| **Time (min)** | **Example 1** | **Example 1 (3M 40°C/75%RH)** | **Comparative Example 2** |
|---|---|---|---|
| 5 | 48.6 | 42.5 | 58.7 |
| 10 | 76.0 | 42.5 | 78.6 |
| 15 | 84.1 | 73.8 | 88.6 |
| 30 | 90.6 | 83.5 | 94.2 |
| 45 | 91.5 | 90.7 | 95.2 |

**Table 8 - Assay Comparison**

| **Lot No** | **Example 1** | **Example 1 (3M 40°C/75%RH)** | **Comparative Example 2** |
|---|---|---|---|
| **%ASSAY** | 99.2 | 99.3 | 102.3 |

**Table 9 - Related Substances**

| **Lot No** | **Example 1** | **Example 1 (3M 40°C/75%RH)** | **Comparative Example 2** |
|---|---|---|---|
| Any Impurity (max) | <LOQ | 0.05% | <LOQ |
| Total Impurities | <LOQ | 0.09% | <LOQ |

As shown in Tables 7 to 9, Example 1 retains a high level of chemical stability. The dissolution and assay results are comparable to the results taken directly after manufacture, before the 3 months. A higher level of impurity is present after 3 months, however, overall, the stability results show Example 1 has good chemical stability.

The related substances analysis was performed using HPLC according to the methodology of Chapter 2.2.29 of the European Pharmacopoeia, 11th Edition (2023).

The polymorphic stability of Example 1 was also investigated using XRD. The stability of Example 1 after 3 months and after 6 months of being stored in high-density polyethylene, with desiccant, and stored at 40 °C and 75% relative humidity was investigated. A placebo sample was prepared with the same qualitative and quantitative (adjusted to 100% final ratio) composition as per Example 1, without the use of the ivacaftor active ingredient. As shown in Figure 1, after both 3 months and 6 months Example 1 maintains its crystallinity and shows good polymorphic stability. Figure 1 also demonstrates the amorphous nature of the ivacaftor used.

XRD was performed according to the methodology of Chapter 2.9.33 of the European Pharmacopoeia, 11th Edition (2023), using a Bruker D2 Phaser Instrument, with a Cu Kα (λ=1.54 Å) source and a LynxEye detector.

The stability tests indicate that the tablets of Example 1 display good chemical and polymorphic stability, comparable to the Comparative Example 2, the commercially available ivacaftor tablet.

The prior art has required solid dispersions or pH dependent polymer to provide a stable composition of amorphous ivacaftor. As such, the inventors surprisingly found that it is possible to manufacture a stable solid composition of amorphous ivacaftor without the need for a solid dispersion, and without the need for a pH dependent polymer. Beneficially, this is considered to negate the downfalls of solid dispersion preparation, and can increases the ease of preparation of amorphous ivacaftor compositions.

The inventors found that a solid composition according to the present invention may exhibit favourable physical attributes sufficient for manufacturing, alongside sufficient stability and/or dissolution when compared to a current pH dependent containing commercial product.

Advantageously, a solid composition according to the present invention avoids the use of certain specific excipients and instead may allow the use of conventional excipients. The present inventors also found that a solid composition according to the present invention lead to acceptable tablets that are within specification, have good process control and/or good scalability.

The inventors also found that a solid composition according to the present invention may be dry granulated using the roller compaction method. This may be beneficial, as the roller compaction process decreases production time compared to other dry granulation methods such as slugging, and so scale-up may be considered easier, with reduced production costs, and improved overall production efficiency. Furthermore, another advantage of roller compaction is that the bulk density of the obtained granule may be increased and/or easily maintained.

## Claims

1. A solid composition of amorphous ivacaftor wherein the composition is free from pH dependent polymers.

2. A solid composition according to claim 1 comprising amorphous ivacaftor in an amount of from about 15 wt.% to about 40 wt.% based on the total weight of the solid composition.

3. A solid composition according to any preceding claim wherein the pH dependent polymer is selected from hypromellose acetate succinate (HPMCAS), cellulose acetate phthalate (CAP), polymethacrylates, hydroxypropyl methyl cellulose phthalates (HPMCP), carboxymethylcellulose (CMC), salts of carboxymethylcellulose, cellulose acetate trimellitate (CAT), hydroxypropylcellulose acetate phthalate (HPCAP), hydroxypropylmethyl-cellulose acetate phthalate (HPMCAP), and methylcellulose acetate phthalate (MCAP), and combinations thereof.

4. A solid composition according to any preceding claim wherein the composition comprises a surfactant, a filler/diluent, a disintegrant, a glidant and/or a lubricant.

5. A solid composition according to any preceding claim wherein the composition comprises:
(a) amorphous ivacaftor in an amount of about 15 wt.% to about 40 wt.%;
(b) a surfactant in an amount of about 0.1 wt.% to about 5 wt.%;
(c) a filler or a diluent in an amount of about 30 wt.% to about 70 wt.%;
(d) a disintegrant in an amount of about 1 wt.% to about 10 wt.%;
(e) a glidant in an amount of about 0.1 wt.% to about 5 wt.%;
(f) a lubricant in an amount of about 0.1 wt.% to about 5 wt.%;
wherein each component is based on the total weight of the solid composition.

6. A solid composition according to any preceding claim wherein the solid composition is coated.

7. A solid composition according to any preceding claim wherein the solid composition is free from solid dispersions.

8. A solid composition according to any preceding claim wherein the solid composition is free-flowing powder, granule, or tablet.

9. A solid composition according to any preceding claim wherein the solid composition is an immediate release composition.

10. A solid composition according to any preceding claim wherein the amorphous ivacaftor is stable at 40 °C and 75% RH for at least 3 months.

11. A process for the preparation of a solid composition according to any one of the proceeding claims.

12. A process according to claim 11 wherein the solid composition is prepared using roller compaction.
